# DEMANDE DE BREVET EUROPEEN

(11) **EP 0 649 653 A1**
(43) Date de publication de la demande: **26.04.1995**
(21) Numéro de dépôt: 94402061.9
(22) Date de dépôt: 15.09.1994
(51) Int. Cl.: A61K 9/70, A61K 47/40

(54) **Utilisation de bêta-cyclodextrines partiellement méthylées comme promoteurs d'absorption dans la préparation de compositions pharmaceutiques pour l'administration transcutanée de principes actifs**

(30) Priorité: 22.09.1993 FR 9311258
(71) Demandeur: ADIR ET COMPAGNIE, F-92415 Courbevoie Cédex (FR)
(72) Inventeur: Cuine, Alain, F-45800 Saint Jean de Braye (FR); de Jong, Hendrik Jan, F-92800 Puteaux (FR)

(57) **Abrégé**

La présente invention concerne l'utilisation de β-cyclodextrines partiellement méthylées comme promoteurs d'absorption dans la préparation de compositions pharmaceutiques pour l'administration transcutanée de principes actifs.

## Description

La présente invention concerne l'utilisation de β-cyclodextrines partiellement méthylées comme promoteurs d'absorption dans la préparation de compositions pharmaceutiques pour l'administration transcutanée de principes actifs.

Les cyclodextrines sont en général connues pour leurs propriétés solubilisantes de nombreuses substances qui permettent d'obtenir des complexes d'insertion solubles dans l'eau. C'est le cas, en particulier, des compositions pharmaceutiques décrites dans le brevet DE 3118218. Il a été montré, d'autre part, que les cyclodextrines, et plus particulièrement la diméthyl-β-cyclodextrine, étaient capables d'augmenter la biodisponibilité d'hormones sexuelles ou de l'insuline lors d'une administration par voie nasale (EP 349091; Pharm. Res., 7, 500-503, 1990 ; Pharm. Res., 8, 588-592, 1991).

Il a été présentement montré que les β-cyclodextrines partiellement méthylées permettaient d'augmenter, de façon surprenante, le passage transdermique de principes actifs.

Ces β-cyclodextrines partiellement méthylées peuvent ainsi être avantageusement utilisées comme promoteurs d'absorption dans des compositions pharmaceutiques pour la libération immédiate ou prolongée de principes actifs par voie transcutanée comme les pommades, les crèmes, les gels, les lotions ou les dispositifs transdermiques.

Parmi les β-cyclodextrines partiellement méthylées, on peut citer les diméthyl-β-cyclodextrines ou de degré de substitution voisin (comme le RAMEB® de WACKER, le PMCD® de RINGDEX ou les diméthyl-β-cyclodextrines commercialisées par CHINOÏN ou NIHON CHEMICS), les triméthyl-β-cyclodextrines, les méthyl-β-cyclodextrines ou les palmitoyl-diméthyl-β-cyclodextrines.

Les compositions pharmaceutiques selon l'invention peuvent également renfermer un ou plusieurs excipients, non toxiques, pharmaceutiquement acceptables. Parmi ces excipients, on peut citer les solvants comme l'éthanol, le polyéthylène glycol, le propylène glycol, le diméthylisosorbide, les tensioactifs comme les Tweens®, les copolymères éthylène/vinylacétate (Elwax®...), les adhésifs comme les adhésifs acryliques, les acides gras saturés ou insaturés, ou leurs dérivés, les polymères, les huiles, les dérivés siliconés, les conservateurs, les antioxydants...

Dans ces compositions pharmaceutiques, les quantités de β-cyclodextrines partiellement méthylées seront comprises entre 0,1 et 50 % de la masse totale de la composition, préférentiellement entre 0,5 et 10 %.

Parmi les principes actifs entrant dans les compositions pharmaceutiques selon l'invention, on peut citer les estrogènes comme l'estradiol, les progestatifs, les médicaments à visée cardiovasculaire, les β-bloquants, les antagonistes calciques, et d'autres agents hypotenseurs, les vasodilatateurs coronariens, les cardiotoniques, les vasodilatateurs périphériques, les vasodilatateurs cérébraux, les antipyrétiques, les analgésiques, les bronchodilatateurs, les antispasmodiques, les antiarthritiques, les antitumoraux, les vitamines, les corticostéroïdes, les hormones polypeptidiques...

Les exemples suivants illustrent l'invention mais ne la limitent en aucune façon.

### Mise en évidence de l'augmentation du passage transcutané de l'estradiol en présence de β-cyclodextrines partiellement méthylées.

Un test in vitro de perméabilité cutanée est réalisé à l'aide d'une cellule de diffusion appelée cellule de Frans (Frans, T.J., 1975, J. Invest. Dermatol. 64, 190-195), dans laquelle un fragment de peau dermatomée à 500 µm de rat "hairless" de 8 semaines sépare le compartiment extérieur et le compartiment récepteur. La cellule est maintenue à 32°C, le milieu récepteur est constitué d'un mélange alcool-eau (70-30 v/v). Des prélèvements sont effectués en continu durant 24 heures à un débit de 0,2 ml/min. Les prélèvements correspondant à 1 heure sont regroupés et dosés.

### Exemple 1:

Composition pharmaceutique sous forme d'une lotion

| | |
|---|---|
| Estradiol | 5 g |
| RAMEB® | 48,875 g |
| Solution aqueuse d'hydroxyde de sodium N | q.s.p. pH 7,4 |
| Eau pour préparation injectable | q.s.p. 1000 ml |

### Exemple 1a :

Lotion témoin sans RAMEB

### Exemple 2:

Composition pharmaceutique sous forme d'un dispositif transdermique

| | |
|---|---|
| Estradiol | 0,5 g |
| Elwax 40W® | 45,6 g |
| Acide oléique | 20,0 g |
| Adhésif acrylique | 29,0 g |
| RAMEB® | 4,9 g |

### Exemple 2a: Dispositif témoin sans RAMEB

### Résultats :

Les résultats obtenus dans le test de perméabilité cutanée avec les compositions pharmaceutiques décrites dans les exemples 1, 1a, 2 et 2a sont regroupés ci-dessous:

| Exemple | Flux (µg/cm²/h) |
|---|---|
| 1 | 2,4 |
| 1a | 1,2 |
| 2 | 3,5 |
| 2a | 1,0 |

Ces résultats montrent que l'utilisation du RAMEB® permet d'obtenir un flux d'estradiol 2 fois plus important pour la lotion et 3,5 fois plus important pour le dispositif transdermique.

### Exemple 3:

Le test de perméabilité cutané tel que décrit précédemment est mis en oeuvre en utilisant un tampon pH 7,4 comme phase réceptrice.

Le passage d'un agoniste dopaminergique, le piribédil monométhanesulfonate, en solution aqueuse à 20 mg/ml est étudié sur de la peau intacte ou sur de la peau prétraitée avec du RAMEB. Le prétraitement consiste en une incubation de la peau dans une solution aqueuse à 500 mg/ml de RAMEB, pendant 24 heures à +4°C.

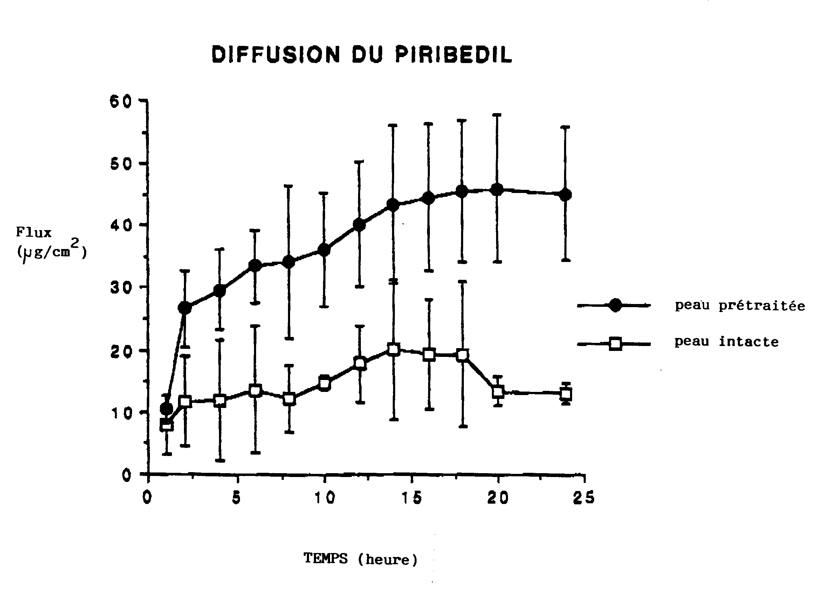

Les résultats représentés sur la figure en annexe montrent une augmentation du flux d'un facteur 4 lorsque la peau est prétraitée avec du RAMEB.

### Exemple 4:

Dans les mêmes conditions que dans l'exemple 3, un test de perméabilité est réalisé avec 2 solutions de chlorhydrate de 1,3,7-triméthyl-8-{3-[4-(diéthylaminocarbonyl)pipérazin-1-yl]prop-1-yl}-3,7-dihydro(1H)purine-2,6-dione (composé A), décrit dans le brevet EP149578, composé hydrophile passant mal la barrière cutanée. L'une des solutions contient du RAMEB, l'autre n'en contient pas. Les résultats regroupés dans le tableau ci-après montrent une augmentation de flux d'un facteur 2 en présence de RAMEB.

| Concentration en composé A (mg/ml) | Concentration en RAMEB (mg/ml) | Flux à 24 h (µg/cm²/h) |
|---|---|---|
| 20 | 0 | 0,49±0,17 |
| 20 | 100 | 0,90±0,25 |

## Revendications

**1/** Utilisation de β-cyclodextrines partiellement méthylées comme promoteurs d'absorption dans la préparation de compositions pharmaceutiques pour l'administration transcutanée de principes actifs.

**2/** Utilisation de β-cyclodextrines partiellement méthylées selon la revendication 1 caractérisée en ce que la composition pharmaceutique est un dispositif transdermique.

**3/** Utilisation de β-cyclodextrines partiellement méthylées selon la revendication 1 caractérisée en ce que les compositions pharmaceutiques sont des pommades, des crèmes, des lotions ou des gels.

**4/** Utilisation de β-cyclodextrines partiellement méthylées selon la revendication 1 caractérisée en ce que la quantité de β-cyclodextrine partiellement méthylée est comprise entre 0,1 et 50 % de la masse totale de la composition.
